# FASCICULE DE BREVET EUROPEEN

(11) **EP 2 872 867 B1**
(45) Date de publication et mention de la délivrance du brevet: **01.04.2020**
(21) Numéro de dépôt: 13747444.1
(22) Date de dépôt: 12.07.2013
(51) Int. Cl.: C12Q 1/24, G01N 1/22, C12M 1/26

(54) **SYSTEME AUTOMATISE DE LYSE DE MICROORGANISMES PRESENTS DANS UN ECHANTILLON, D'EXTRACTION ET DE PURIFICATION DES ACIDES NUCLEIQUES DESDITES MICROORGANISMES AUX FINS D'ANALYSE**
AUTOMATISIERTES SYSTEM ZUR LYSE VON MIKROORGANISMEN IN EINER PROBE SOWIE ZUR EXTRAKTION UND REINIGUNG DER NUKLEINSÄUREN AUS DEN BESAGTEN MIKROORGANISMEN ZUR ANALYSE
AUTOMATED SYSTEM FOR THE LYSIS OF MICROORGANISMS PRESENT IN A SAMPLE, FOR EXTRACTION AND FOR PURIFICATION OF THE NUCLEIC ACIDS OF SAID MICROORGANISMS FOR PURPOSES OF ANALYSIS

(30) Priorité: 13.07.2012 FR 1256758
(43) Date de publication de la demande: 20.05.2015
(73) Titulaire: Biomérieux, 69280 Marcy l'Étoile (FR)
(72) Inventeur: GUY, Michel, F-38000 Grenoble (FR); ROSTAING, Hervé, F-38420 Le Versoud (FR)
(86) Numéro de dépôt international: PCT/FR2013/051681
(87) Numéro de publication internationale: WO 2014/009673

(56) Documents cités:
- WO-A2-2009/001010
- WO-A2-2010/067019
- WO-A2-2010/133776
- US-A1- 2010 112 667

## Description

Le domaine technique de la présente invention est celui de l'analyse biologique. Plus particulièrement, la présente invention concerne en premier lieu un dispositif de lyse des microorganismes présents dans un échantillon environnemental ou clinique, d'extraction et de purification des acides nucléiques desdits microorganismes. L'invention concerne en outre un système automatisé de lyse des microorganismes d'extraction et de purification des acides nucléiques desdits microorganismes, aux fins d'analyse.

On observe, depuis plusieurs années, une recrudescence des infections nosocomiales au sein des hôpitaux. Ces infections s'expliquent par la contamination des personnes hospitalisées, et donc par définition immunodéprimées, par des microorganismes pathogènes présents dans la sphère environnementale hospitalière et non détruits malgré le soin toujours important apporté à la désinfection des outils et des surfaces et au traitement de l'air. Eu égard à ces cas de plus en plus fréquents de contaminations microbiologiques environnementales, la mise au point de dispositifs et de méthodes permettant d'améliorer et de faciliter les contrôles environnementaux est devenu un enjeu majeur pour les professionnels de santé.

Au-delà du problème des infections nosocomiales, le contrôle des conditions environnementales est également devenu depuis plusieurs années, un souci récurrent dans le milieu industriel, en particulier les industries agro-alimentaires, les industries pharmaceutiques ou cosmétiques. Dans les industries agro-alimentaires, on sait les conséquences désastreuses sur la santé des consommateurs, que peut avoir la contamination des produits, voire de matières premières, par un microorganisme pathogène. En effet, les toxi-infections alimentaires, dues à des bactéries telles que celles du genre *Listeria* ou *Salmonella* sont aujourd'hui monnaie courante. Le contrôle de la qualité de l'air est également un processus-clé dans la démarche qualité des industries pharmaceutiques ou cosmétiques.

Par ailleurs, ces contrôles doivent répondre à un niveau d'exigence toujours plus important, de par une règlementation toujours plus stricte.

Parmi les outils à disposition des professionnels de santé ou des industriels pour la réalisation de contrôles environnementaux, les aéro-bio-collecteurs sont des solutions de choix pour la détection des microorganismes de l'air. Ces dispositifs sont placés aux endroits appropriés dans les lieux où l'on souhaite mesurer l'aéro-bio-contamination. Ils sont généralement constitués d'un collecteur d'air couplé à un milieu de culture. L'air collecté par le collecteur d'air entre en contact avec le milieu de culture ; milieu de culture sur lequel viennent se déposer les microorganismes éventuellement contenus dans l'air collecté. Le milieu de culture est ensuite récupéré et placé à l'étuve pour favoriser la croissance des microorganismes. Il est ainsi possible de détecter et d'identifier lesdits microorganismes par des techniques traditionnelles de microbiologie.

Ces dispositifs présentent néanmoins un inconvénient majeur qui est lié à la technologie utilisée. Cet inconvénient est le temps nécessaire pour obtenir le résultat d'analyse. En effet, l'utilisation des techniques traditionnelles de microbiologie, en particulier de bactériologie, implique le respect de temps d'incubation nécessaires à la croissance cellulaire, voire des phases de réensemencement sur des milieux de culture spécifiques pour permettre l'identification. Il s'ensuit que le temps nécessaire pour obtenir un résultat est relativement long, voire trop long, quand on cherche à détecter et identifier un organisme pathogène, responsable d'une infection nosocomiale ou d'une toxi-infection alimentaire.

Un autre inconvénient de ce type de dispositif est que l'utilisation de milieux de culture, s'il permet de discriminer entre les genres et espèces bactériennes, ne permet généralement pas de discriminer les souches d'une même espèce bactérienne. Or, l'on sait que la pathogénicité d'un microorganisme peut varier de manière significative selon la souche considérée.

Par ailleurs, ce type de dispositif présente comme inconvénient de ne pas permettre la mise en évidence des microorganismes présents dans l'air, viables mais non cultivables.

Ces dispositifs ont également pour inconvénient le séchage des surfaces nutritives au fur et à mesure de la collection.

Il existe par ailleurs des dispositifs destinés à la récupération des particules présentes dans l'air, en particulier des microorganismes. Le document GB-2 254 024 décrit ainsi un dispositif pour collecter les particules contenues dans l'air dont le principe est basé sur l'effet cyclone. Si un tel dispositif se montre approprié à la collecte des particules contenues dans l'air, y compris les microorganismes, il n'est en aucun cas étudié pour traiter l'échantillon ainsi obtenu, en particulier pour réaliser l'extraction du matériel génétique destiné à être utilisé pour l'analyse.

De manière plus générale, les techniques les plus pertinentes en termes d'identification de microorganismes et/ou de rapidité de rendu des résultats, que ce soit vis-à-vis d'échantillons cliniques ou environnementaux, sont sans nul doute les techniques de diagnostic moléculaire. Ces techniques basées sur l'analyse du matériel génétique des microorganismes, et en particulier de certaines séquences spécifiques d'intérêt, permettent d'obtenir une identification très pointue des microorganismes en un temps record, puisqu'elles permettent de s'affranchir des étapes de culture.

Néanmoins, l'utilisation de telles techniques présente un certain nombre de limites, parmi lesquelles la plus importante est la quantité potentiellement limitée de microorganismes présents dans l'air et donc récupérable pour réaliser l'analyse. En effet, on sait que les prélèvements environnementaux, mais aussi certains prélèvements cliniques sont relativement pauvres en microorganismes. Il s'ensuit que la quantité de matériel génétique obtenue à partir de cette matière première est faible. Les performances de la technique utilisée pour extraire les acides nucléiques, en termes de rendement, deviennent alors un paramètre crucial.

Par ailleurs, la plupart des techniques existantes de lyse de microorganismes, ne sont pas génériques vis-à-vis de l'ensemble des microorganismes et/ou nécessitent l'intervention de personnel qualifié pour prendre en charge les étapes manuelles.

Le document WO-A-2005/038025 décrit une méthode d'extraction d'acides nucléiques de microorganismes prélevés notamment dans l'air. Cette méthode consiste dans la mise en œuvre de trois méthodes de lyse différentes, à savoir une lyse chimique, une lyse par choc thermique et une lyse mécanique. Si une telle méthode permet sans nul doute d'optimiser le rendement d'extraction des acides nucléiques et donc d'augmenter la quantité de matériel génétique disponible pour l'analyse, il n'en demeure pas moins que ce rendement reste dépendant de la quantité de microorganismes récupérés. Or, rien dans ce document n'est décrit pour optimiser la récupération desdits microorganismes.

Le document US-A-5,707,861 décrit un dispositif permettant de désintégrer des cellules vivantes du type microorganismes. Ce dispositif permet de lyser les cellules en utilisant à la fois des billes de verre mais également l'effet de vibration dû à l'espace existant entre les tubes contenant les microorganismes et les trous du support portant lesdits tubes. Ainsi, un tel dispositif permet d'optimiser la lyse de cellules et donc d'optimiser l'extraction du matériel génétique. Un tel dispositif et la méthode mise en œuvre par ce dernier présentent les mêmes limites que celles exposées précédemment, à savoir qu'ils restent dépendants de la quantité de microorganismes récupérés. Par ailleurs, ils présentent comme inconvénient supplémentaire de devoir effectuer subséquemment une étape de concentration des acides nucléiques afin de les isoler des débris cellulaires. Ils nécessitent enfin de récupérer manuellement les acides nucléiques, à l'issue de l'étape de concentration.

Ces problèmes se posent également avec le dispositif décrit dans le document US-5,567,050.

Des systèmes plus intégrés ont également été décrits. Ainsi, le document WO-A-2004/018704 décrit un dispositif, et une méthode associée utilisant la technique d'amplification PCR (Polymerase Chain Reaction), pour collecter des microorganismes dans l'air et les identifier. Ce système est spécialement adapté à la lutte contre les tentatives d'attentats par contamination biologique dans les centres de tri postal. Ce système se compose d'un dispositif de collecte d'air placé le long du circuit de transport du courrier, un dispositif de filtration/séparation des particules par effet cyclone, un dispositif de concentration/récupération des particules dans un échantillon liquide, un dispositif de transfert d'une fraction de l'échantillon dans une cartouche d'analyse par PCR GeneXpert™ de la société Cepheid en Californie. La cartouche est ensuite transférée manuellement dans un automate d'analyse biologique indépendant à fin d'identification du ou des microorganismes collectés dans l'air.

Si ce système permet de résoudre un bon nombre de problèmes techniques liés aux dispositifs et procédés décrits précédemment, il présente néanmoins des inconvénients majeurs. Le premier de ces inconvénients est que le système de traitement de l'échantillon (collecte, séparation, concentration/récupération) préalablement au transfert dans la cartouche d'analyse est relativement complexe et coûteux. Un deuxième inconvénient est que les microorganismes collectés sont récupérés dans un échantillon liquide dont seule une fraction est analysée. Ce qui signifie que le risque de ne pas récupérer la totalité des microorganismes et donc la totalité des acides nucléiques est très important, limitant fortement la pertinence de l'analyse. Par ailleurs, malgré sa complexité, ce système nécessite le transfert manuel de la cartouche dans l'automate d'analyse GeneXpert™.

La Demanderesse a déjà déposé une demande de brevet WO-A-2009/001010 qui concerne en premier lieu une cartouche, susceptible d'être positionnée à l'intérieur d'un moyen de collecte d'air et à recevoir un moyen de récupération des acides nucléiques, ladite cartouche de forme sensiblement cylindrique, comportant une zone de rétention des microorganismes, ladite zone de rétention comportant des moyens de lyse de microorganismes. Cette invention concerne en outre un dispositif de collecte de microorganismes contenus dans l'air et un dispositif de lyse de microorganismes.

La Demanderesse a également déjà déposé une demande de brevet WO-A-2010/067019 qui concerne entre autres un dispositif de collecte de microorganismes contenus dans l'air, ledit dispositif comportant :
- un module de collecte d'air, comprenant :
   i. un élément supérieur comportant un conduit d'admission de l'air permettant l'entrée d'un flux d'air à l'intérieur dudit module, ledit conduit disposant à sa base, de moyens de perturbation du flux d'air,
   ii. un élément inférieur comportant des moyens d'évacuation de l'air permettant la sortie du flux d'air créé
   lesdits éléments supérieur et inférieur pouvant être solidarisés l'un à l'autre de sorte que le flux d'air puisse être créé à l'intérieur dudit module de collecte d'air ;
- une cartouche, de forme sensiblement cylindrique, comportant une zone de rétention des microorganismes, ladite zone de rétention comportant des moyens de lyse de microorganismes, ladite cartouche étant positionnée à l'intérieur dudit module de collecte d'air.

Avec ces deux nouvelles solutions, il est indéniable que le processus est amélioré avec une lyse universelle, à la fois efficace pour des prélèvements environnementaux et cliniques, pour une grande diversité de microorganismes, qu'il s'agisse de bactéries, de virus ou encore de champignons, éventuellement à l'état végétatif ou sous forme de spores.

De plus il y a une assez forte concentration des micro-organismes, le système est compatible avec différentes analyses en aval (de type PCR, étalement sur boîte, etc.) et la lyse mécanique est intégrée.

Toutefois, il n'est pas dans la nature humaine de se contenter d'un système qui fonctionne bien quand on peut trouver une solution encore plus optimale. Ainsi dans ces deux cas de figure, la collecte est toujours limitée par le volume de fluide, gazeux ou liquide, qui va en contact avec le milieu de récupération, en général un milieu de culture. Le flux de fluide va toujours venir frapper ce milieu au même endroit, ce qui va entraîner :
- soit la saturation dudit milieu en un endroit précis sans que le reste ne soit utilisé,
- soit la dégradation de ce milieu car il est tout le temps frappé par un gaz ou un liquide au(x) même(s) endroit(s), ce qui entraine un dessèchement dans le cas d'un gaz et une liquéfaction de la surface du milieu dans le cas d'un liquide. Dans les deux cas et notamment dans le cas du séchage de la gélose, il y aura une diminution très nette du rendement de capture en fonction du temps.

De plus le mode de capture par impaction est très peu efficace pour les petites particules d'un diamètre inférieur à 1 µm. Autre problème, il y a un risque d'entraînement d'une partie du milieu de récupération (billes avec du glycérol) par le flux d'air. Ainsi, cette projection de fluide et son rebond sur ledit milieu peut engendrer des éclaboussures qui sont autant de risques de contamination.

On connait également du document WO2010/133776 un dispositif de collecte et de concentration de cibles biologiques ou chimiques en vue de sa détection. Ce dispositif comprend une chambre de réaction dans laquelle sont placées des billes magnétiques fonctionnalisées, un système d'agitation par ultrasons, un champ magnétique pouvant être activé (position ON) ou inactivé (position OFF), et un système de déplacement de fluide pour alimenter et décharger une partie ou la totalité du contenu de la chambre de réaction. L'invention concerne également un procédé de capture de cibles biologiques ou chimiques mettant en œuvre ledit dispositif.

En outre, on connaît du document US20100112667 une cartouche micro-fluidique pour l'isolation de molécules biologiques avant une chambre de capture contenant des supports solides fonctionnalisés maintenus à l'état fluidisé permet de réduire les pertes de charge et la formation de bulles lors de l'extraction micro-fluidique. La cartouche peut comprendre une chambre de lyse par champ électrique et/ou une chambre de lyse chimique. La chambre de lyse par champ électrique peut comprendre une structure électriquement isolante disposée entre deux électrodes planes opposées.

La présente invention a pour objectif de proposer un appareil qui répond à ces inconvénients ci-dessus énumérés.

Pour cela l'invention propose un dispositif de collecte de microorganismes contenus dans un fluide, le dispositif comportant :
- un module réactionnel renfermant un ensemble de billes,
- au moins un conduit d'admission du fluide permettant l'entrée d'un flux de fluide à l'intérieur du module,
- au moins un conduit d'évacuation du fluide permettant la sortie du flux dudit fluide ayant traversé l'intérieur dudit module,
- des moyens de rétention des billes à l'intérieur du module,
- au moins un canal d'admission d'au moins un liquide réactionnel,
- au moins un canal d'évacuation d'au moins un liquide réactionnel,
les canaux d'admission et d'évacuation du liquide réactionnel et les conduits d'admission et d'évacuation du fluide étant positionnés de la manière suivante au niveau du module:
- les conduits d'admission et d'évacuation du fluide se faisant face tout en enserrant le module, afin de maximiser les contacts entre le fluide et les billes,
- les canaux pour le ou les liquides réactionnels étant placés respectivement à deux extrémités opposées du module, et
- les canaux pour le ou les liquides réactionnels sont positionnés selon un plan, et les conduits d'admission et d'évacuation du fluide sont positionnés selon un axe, et que l'axe est sensiblement perpendiculaire au plan.

Selon un premier mode de réalisation, le dispositif, selon l'invention, comprend une enceinte de confinement étanche isolant fluide et liquides réactionnels de l'extérieur.

Selon une première variante de réalisation du dispositif, si le fluide est un gaz, l'ensemble de billes est constitué de billes recouvertes de glycérol.

Selon une seconde variante de réalisation du dispositif, si le fluide est un liquide, le module contient un filtre s'étendant sensiblement dans le plan formé par les canaux pour le ou les liquides réactionnels, le(s) canal(canaux) d'admission étant situé (s) d'un côté du filtre avec l'ensemble de billes et le (s) canal(canaux) d'évacuation étant situé(s) de l'autre côté dudit filtre en l'absence de billes. Cette disposition permet ainsi au fluide de rentrer en contact avec les billes puis de circuler au travers du filtre, afin de séparer par exemple d'éventuels résidus cellulaires sans intérêt ou inhibiteurs, tels que protéines, membranes.

Dans un mode de réalisation particulier, la forme du module si on le coupe :
- selon le plan formé par les canaux d'admission et d'évacuation du liquide réactionnel, et
- perpendiculairement à l'axe formé par les conduits d'admission et d'évacuation du fluide
comprend au moins une partie rectilignes et/ou au moins une partie en arc comprise(s) entre deux extrémités. Le(s) canal (canaux) d'admission du liquide réactionnel étant situé(s) à une extrémité du module et le(s) canal (canaux) d'évacuation du liquide réactionnel étant situé(s) à l'autre extrémité du module, de sorte que le liquide réactionnel traverse entièrement le lit de bille entre le moment où il est admis dans le(s) canal(canaux) d'admission du liquide réactionnel et le moment où il est évacué par le(s) canal(canaux) d'évacuation du liquide réactionnel. Selon ce dernier mode ou variante, le module à une section de quadrilatère si on le coupe selon un plan perpendiculaire au plan formé par les canaux d'admission et d'évacuation du liquide réactionnel, et passant par l'axe formé par les conduits d'admission et d'évacuation du fluide.

Dans un mode de réalisation particulier, le module à une forme de « C » si on le coupe :
- selon le plan formé par les canaux d'admission et d'évacuation du liquide réactionnel, et
- perpendiculairement à l'axe formé par les conduits d'admission et d'évacuation du fluide

Une forme de « C » comprend au moins une partie en arc correspondant à une partie de la circonférence d'un cercle ou d'une parabole reliant deux points. Les extrémités du « C » étant les deux zones terminales du module, de sorte que le (s) canal (canaux) d'admission du liquide réactionnel étant situé(s) à une extrémité du module et le(s) canal (canaux) d'évacuation du liquide réactionnel étant situé(s) à l'autre extrémité du module, le liquide réactionnel traverse entièrement le lit de bille entre le moment où il est admis dans le(s) canal (canaux) d'admission du liquide réactionnel et le moment où il est évacué par le(s) canal(canaux) d'évacuation du liquide réactionnel.

Selon ce dernier mode ou variante, le module à une forme de quadrilatère si on le coupe selon un rayon passant par le centre du « C » et coupant ledit « C ».
Toujours selon ce dernier mode ou variante, le(s) canal(canaux) d'admission du liquide réactionnel est(sont) situé(s) à une extrémité du « C » de la forme du module, et que le(s) canal(canaux) d'évacuation du liquide réactionnel est(sont) situé(s) à l'autre extrémité du « C ».

Quel que soit le mode ou la variante de réalisation, les billes ont un diamètre compris entre 200 et 600 µm et les moyens de rétention desdites billes à l'intérieur du module sont constitués de grilles, dont les pores ont un diamètre inférieur aux billes et compris entre 100 et 500 µm.

Quel que soit le mode ou la variante de réalisation, il comprend un autre module réactionnel permettant le traitement des microorganismes collectés, le module contenant :
- des moyens de séparation des résidus cellulaires sans intérêt ou inhibiteurs, tels que protéines, membranes, et/ou
- des moyens d'amplification des acides nucléiques collectées et séparés des inhibiteurs, et/ou
- des moyens de détection des amplicons ainsi générés.

La présente invention concerne également un procédé de collecte de microorganismes contenus dans un fluide, qui consiste à :
- introduire du fluide suspecté de contenir des microorganismes via au moins un conduit d'admission du fluide,
- traverser un lit de billes où les microorganismes sont immobilisés,
- évacuer le fluide débarrassé desdits microorganismes via au moins un conduit d'évacuation dudit fluide,
- introduire au moins un liquide réactionnel via au moins un canal d'admission de liquide(s) réactionnel(s),
- traverser le lit de billes où les microorganismes sont immobilisés afin de mettre lesdits microorganismes en suspension, et
- évacuer ce liquide transportant les microorganismes via au moins un canal d'évacuation de liquide(s) réactionnel(s), les conduits d'admission et d'évacuation du fluide se faisant face tout en enserrant le lit de billes, les canaux d'admission et d'évacuation de liquide(s) réactionnel(s) étant positionnés de manière à accéder à l'ensemble des billes.

Selon un mode de réalisation, lorsque le fluide est un gaz, les billes sont recouvertes de glycérol.

Selon un mode de réalisation, lorsque le fluide est un liquide, les billes sont recouvertes d'un revêtement de polymère.

Selon un mode de réalisation, lorsque le liquide réactionnel traverse le lit de billes, lesdites billes sont agitées par un moyen d'agitation extérieur et entrent en contact ce qui permet de détruire ou de lyser les membranes des microorganismes, rendant les acides nucléiques accessibles et prêts à être évacués.

Selon un mode de réalisation, une quantité définie de liquide réactionnel est admise dans le(s) canal(canaux) d'admission du liquide réactionnel et n'est pas évacuée par le(s) canal(canaux) d'évacuation du liquide réactionnel pendant la lyse de telle sorte que les microorganismes sont lysés pendant une durée définie en présence d'une quantité définie de liquide réactionnel. Cette durée étant préférentiellement comprise entre 5 et 30 minutes et cette quantité de liquide étant préférentiellement comprise entre 100µl et 1 ml, plus préférentiellement de 500µl.

Selon une variante de ce mode de réalisation, l'agitation des billes est réalisée par l'utilisation d'ultrasons.

Quel que soit le mode ou la variante de réalisation, le liquide chargé de tout ou partie des microorganismes est évacué vers un autre module réactionnel du dispositif permettant le traitement des microorganismes collectés, le traitement consistant à :
- séparer les résidus cellulaires sans intérêt ou inhibiteurs, tels que protéines, membranes, et/ou
- amplifier les acides nucléiques collectées, et/ou
- détecter les amplicons ainsi générés.

Quel que soit le mode ou la variante de réalisation, une première possibilité pour ce procédé se caractérise en ce que :
- le fluide testé est constitué par un gaz, (par exemple de l'air)
- le gaz traverse le lit de billes, billes qui sont recouvertes de glycérol, où les microorganismes sont immobilisés, et
- l'on introduit au moins un liquide réactionnel afin de liquéfier le glycérol et de mettre lesdits microorganismes en suspension.
Quel que soit le mode ou la variante de réalisation, une seconde possibilité pour ce procédé se caractérise en ce que :
- le fluide testé est constitué par un liquide,
- le liquide testé traverse le lit de billes puis un filtre avant d'être évacué, débarrassé des microorganismes qu'il contenait, les microorganismes sont immobilisés, et
- l'on introduit au moins un liquide réactionnel afin de mettre lesdits microorganismes en suspension.
Selon l'une quelconque de ces deux possibilités, le liquide est filtré entre l'évacuation via au moins un canal d'évacuation de liquide(s) réactionnel(s) et le module permettant l'élimination des inhibiteurs et/ou l'amplification et/ou la détection.

Par échantillon fluidique, on entend tout échantillon gazeux ou liquide susceptible de contenir des microorganismes. Il peut s'agir d'un échantillon d'origine humaine ou animale. Cet échantillon peut être par exemple, de l'urine, du sang total, du plasma ou tout autre liquide corporel ou de l'air expiré. L'échantillon peut être d'origine alimentaire tel qu'une boisson. Il peut être également d'origine environnementale, tel que de l'eau ou de l'air confiné. Par ailleurs, l'échantillon liquide peut être également un liquide dit de transfert, dans lequel des éventuels microorganismes contenus sur un dispositif de prélèvement de surface, du type écouvillon tel que ceux commercialisés par la société COPAN, sous la dénomination « flocked SWABS », ont été re-suspendus par agitation dudit écouvillon dans ledit liquide de transfert.

Les microorganismes sont pris dans le groupe comprenant les bactéries, les virus, les levures, les moisissures, les parasites.

Les échantillons à partir desquels sont isolés les microorganismes sont d'origine environnementale. Ainsi, il peut s'agir d'un échantillon d'air ou de liquide, tel que de l'eau ; des prélèvement de surface. Les échantillons peuvent être également d'origine clinique, à savoir tout échantillon d'origine humaine ou animale, apte à faire l'objet d'une analyse pour la rechercher et l'identification d'un microorganisme, éventuellement pathogène.

La présence des acides nucléiques cibles peut être mise en évidence par la visualisation de réactions d'hybridation. On entend par réaction d'hybridation toute réaction entre un acide nucléique de capture et un acide nucléique cible isolé ou généré par une étape de transcription, de transcription inverse ou d'amplification de type, par exemple, NASBA (pour Nucleic Acid Sequence Based Amplification en anglais) ou PCR.

On entend par acide nucléique, les oligonucléotides, les acides désoxyribonucléiques et les acides ribonucléiques, ainsi que leurs dérivés. Le terme oligonucléotide désigne un enchaînement d'au moins deux nucléotides (désoxyribonucléotides ou ribonucléotides, ou les deux), préférentiellement au moins cinq, préférentiellement au moins huit, et encore plus préférentiellement au moins quatorze, qu'ils soient naturels ou modifiés, susceptibles de s'hybrider, dans des conditions appropriées d'hybridation, avec un autre oligonucléotide au moins partiellement complémentaire.

Par nucléotide modifié, on entend par exemple un nucléotide comportant une base modifiée et/ou comportant une modification au niveau de la liaison internucléotidique et/ou au niveau du squelette. A titre d'exemple de base modifiée, on peut citer l'inosine, la méthyl-5-désoxycytidine, la diméthylamino-5-désoxyuridine, la diamino-2,6-purine et la bromo-5-désoxyuridine.

Pour illustrer une liaison internucléotidique modifiée, on peut mentionner les liaisons phosphorothioate, N-alkylphosphoramidate, alkylphosphonate et alkylphosphodiester.

Les alpha-oligonucléotides tels que ceux décrits dans la demande de brevet FR-A-2.607.507, les LNA tels que phosphorothioate-LNA and 2'-thio-LNA décrits dans Bioorganic & Medicinal Chemistry Letters, Volume 8, Issue 16,18 August 1998, pages 2219-2222, et les PNA qui font l'objet de l'article de M. Egholm et al., J. Am. Chem. Soc. (1992), 114,1895- 1897, sont des exemples d'oligonucléotides constitués de nucléotides dont le squelette est modifié.

La visualisation des réactions d'hybridation peut être effectuée par tout moyen de détection, tels que des moyens directs ou indirects.

Dans le cas de la détection directe, c'est-à-dire sans l'intermédiaire d'un marquage, on observe les réactions d'hybridation par résonance plasmon ou par voltamétrie cyclique sur une électrode portant un polymère conducteur.

Dans le cas de la détection indirecte, c'est-à-dire par l'intermédiaire d'un marquage, le marquage peut être effectué soit directement sur les acides nucléiques cibles, soit par l'intermédiaire d'un partenaire de liaison spécifique desdits acides nucléiques préalablement marqués.

On entend par partenaire de liaison spécifique des acides nucléiques cibles tout partenaire capable de se lier avec l'acide nucléique cible et on donnera à titre d'exemples les acides nucléiques, les oligonucléotides ou polynucléotides et les substrats enzymatiques.

Par marquage, on entend la fixation d'un marqueur capable de générer directement ou indirectement un signal détectable. Une liste non limitative de ces marqueurs consiste en : les enzymes qui produisent un signal détectable par exemple par électrochimie, colorimétrie, fluorescence, luminescence, enzymes comme la péroxydase de Raifort (HRP), la phosphatase alcaline (PAL), la beta-galactosidase, la glucose-6-phosphate déshydrogénase ; les inhibiteurs d'enzymes ; les co-facteurs d'enzymes ; les particules telles que les particules en or, les latex magnétiques, les liposomes ; les chromophores comme les composés, luminescents, colorants, les molécules radioactives comme le ³²P, le ³⁵S ou le ¹²⁵I, les molécules fluorescentes telles que la fluorescéine, la rhodamine, les Alexa®, l'umbelliférone, le luminol ou les phycocyanines. Dans le cas de la fluorescence, il peut s'agir du produit fluorescent d'une réaction enzyme-substrat, d'une combinaison fluorophore-quencher, d'une extinction de fluorescence ou de tout autre système basé sur des propriétés de fluorescence.

Des systèmes indirects peuvent être aussi utilisés, comme par exemple par l'intermédiaire d'un autre couple ligand/antiligand. Les couples ligand/antiligand sont bien connus de l'homme du métier, et on peut citer par exemple les couples suivants : biotine/streptavidine, sucre/lectine, polynucléotide/complémentaire du polynucléotide. Dans ce cas, c'est le ligand qui porte l'agent de liaison. L'antiligand peut être détectable directement par les marqueurs décrits au paragraphe précédent ou être lui-même détectable par un ligand/anti-ligand.

Ces systèmes de détection indirects peuvent conduire, dans certaines conditions, à une amplification du signal. Cette technique d'amplification du signal est bien connue de l'homme du métier, et l'on pourra se reporter aux demandes de brevet antérieures FR-A-2 781 802 et WO-A-95/08000 de la demanderesse ou à l'article J. Histochem. Cytochem. 45 : 481-491, 1997.

Le marquage préalable des acides nucléiques cibles peut être effectué par incorporation directe ou indirecte de marqueur par une polymérase, par une kinase, de façon aléatoire ou spécifique, aux extrémités ou par incorporation « à l'intérieur » de la molécule.

Le marquage des partenaires de liaison spécifiques des analytes cibles est largement connu de l'homme du métier et est décrit par exemple par Greg T. Hermanson dans Bioconjugate Techniques, 1996, Academic Press Inc, 525B Street, San Diego, CA92101 USA.

Selon le type de marquage du conjugué utilisé, comme par exemple en utilisant une enzyme, l'homme du métier ajoutera des réactifs permettant la visualisation du marquage. Cette étape correspond à la révélation. Elle est précédée de l'utilisation d'un tampon de lavage qui permet d'éliminer les fractions d'analytes ou d'éléments non engagés dans la réaction, ou faiblement ou non spécifiquement liés, afin de limiter le bruit de fond.

Les buts et avantages du dispositif selon la présente invention seront mieux compris à la lumière de l'exemple nullement limitatif qui suit, en référence aux figures, dans lesquelles :
- La Figure 1 représente une vue éclatée du dessus de la carte ou cartouche utilisée pour collecter et lyser les microorganismes avec tous les éléments qui lui permettent de fonctionner, selon un mode particulier de réalisation de l'invention.
- La Figure 2 représente une vue du dessus en perspective de la carte principale formant l'âme du dispositif selon le mode de réalisation de l'invention présenté à la figure 1.
- La Figure 3 représente une vue du dessous en perspective de la carte principale, toujours selon le mode de réalisation de l'invention présenté à la figure 1.
- La Figure 4 représente une vue de détail de la figure 2 au niveau du lit de billes et de la grille les retenant dans le plan en contact avec au moins un conduit d'admission du fluide et permettant la capture des microorganismes. Les deux extrémités opposées du module en forme de « C » sont connectées fluidiquement aux canaux d'admission et d'évacuation du liquide réactionnel.
- La Figure 5A est un schéma de principe de la partie filtrante de la carte principale où se trouvent présentes les billes lorsque les plaques de confinement sont fermées.
- La Figure 5B est un schéma de principe similaire à celui de la Figure 5A, mais dans lequel les plaques de confinement sont ouvertes.
- La Figure 6 est la reprise d'un détail A de la figure 5B mais dans lequel le mouvement du fluide au sein des billes de capture et le mouvement des plaques de confinement sont mis en exergue.
- La Figure 7 est identique à la figure 6 mais se focalise sur le nouveau mouvement des plaques de confinement et le début du mouvement du liquide réactionnel au sein des billes de capture.
- La Figure 8 est identique à la figure 7 et montre bien le mouvement du fluide au sein desdites billes de capture.
- La Figure 9 est identique à la figure 8 mais précise l'extraction des acides nucléiques.
- Les Figures 10A à 10D montrent l'efficacité du remplissage de la chambre où les microorganismes sont retenus par les moyens de rétention au fil du temps.
- La Figure 11 représente un graphe démontrant l'efficacité de la lyse au sein d'un dispositif selon l'invention.

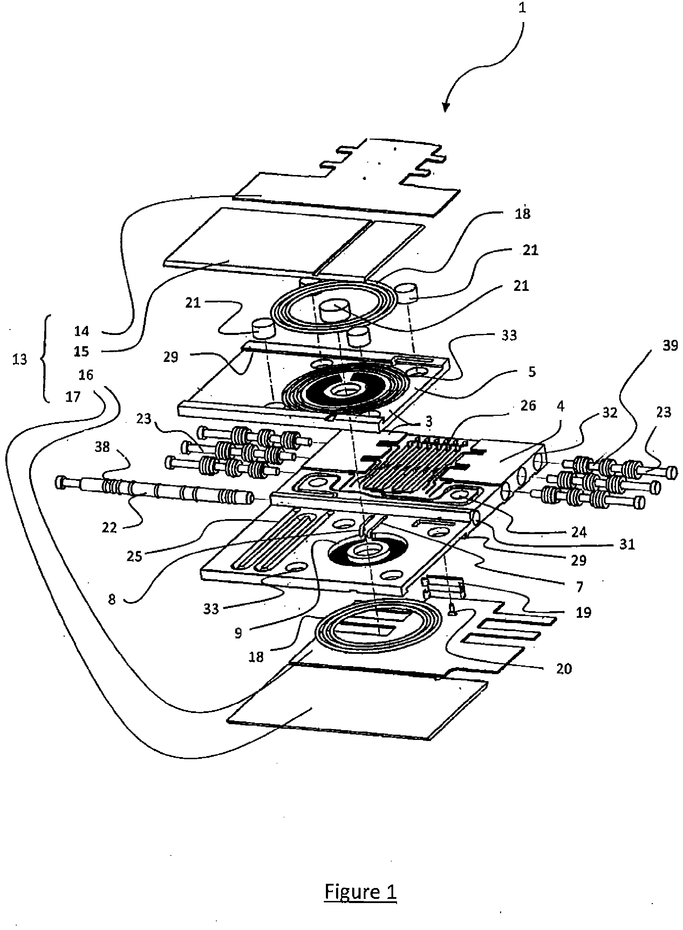

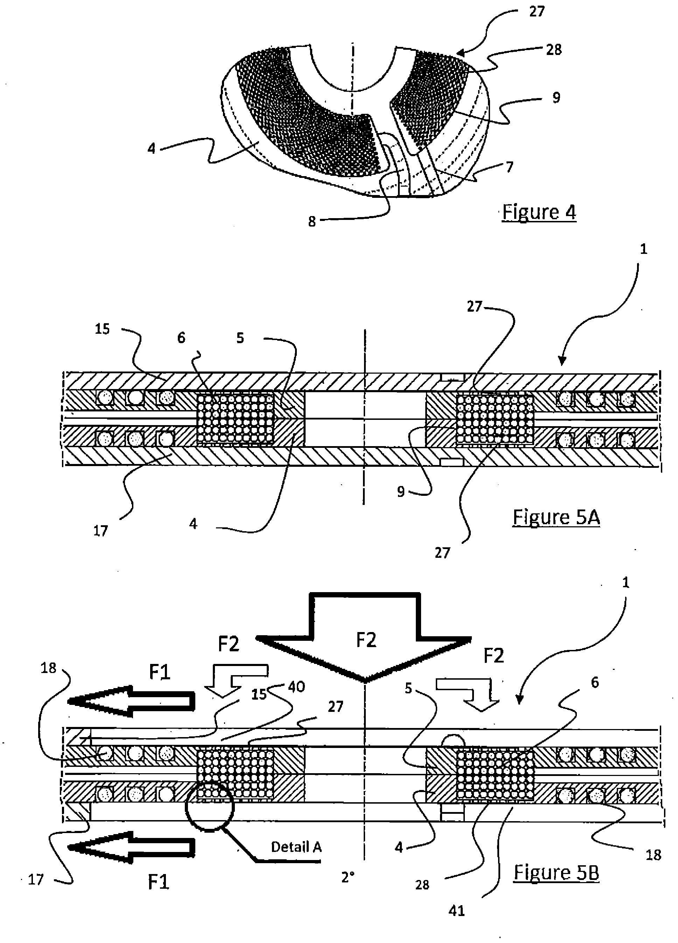

La présente invention concerne un dispositif de collecte 1 de microorganismes. Sur la Figure 1 on peut voir une vue éclatée d'un mode de réalisation présenté par la demanderesse dans lequel le dispositif 1 pour la collecte de microorganismes est présenté sans la présence d'aucun fluide 2 ou liquide 12. Ledit dispositif de collecte 1 comporte essentiellement deux grandes parties, une carte principale 4 située en position inférieure par rapport à une carte secondaire 5, elle, en position supérieure. La carte principale 4 ayant une forme de « L », la carte secondaire 5 s'intègre dans cette forme en « L » pour donner une forme générale de parallélépipède. Ces cartes principale 4 et secondaire 5 sont destinées à être mises en contact l'une de l'autre. De ce fait elles vont pouvoir former un moyen 9 de rétention de billes 6, essentiellement constitué par une cavité présente dans la partie horizontale de la forme en « L », cette cavité 9 étant fermée par la face inférieure de la carte secondaire 5 en contact avec la carte 4. Les deux cartes principale 4 et secondaire 5 forment une fois assemblées le module réactionnel 3. Néanmoins, le moyen de rétention 9 n'est pas simplement constitué par les bords de ce module réactionnel 3 mais comporte également une ou des grilles 27, non représentées sur la figure 1, permettant le passage de fluide 2 ou des liquides 12 sans pour autant que les billes 6, non représentées également, ne puissent sortir du module réactionnel 3. Sur la grille 27 visible sur la Figure 4, le diamètre des trous 28 de la grille 27 dépend du diamètre des billes 6 utilisées. Les trous 28 de la grille 27 doivent être d'un diamètre inférieur au diamètre des billes de verre. Dans un mode préférentiel de réalisation, les trous 28 de la grille 27 doivent être d'un diamètre significativement inférieur au diamètre des billes de verre, « significativement » veut dire par exemple que les billes 6 ont un diamètre de 400 à 600 µm et que la grille 27 a des trous 28 de 200 à 250 µm.

L'ensemble des deux cartes 4 et 5 ainsi que des conduits d'admission et d'évacuation du fluide 40 et 41 et canaux d'admission et d'évacuation du liquide réactionnels 7 et 8, présentés sur la Figure 4,sont confinés à l'aide d'une enceinte de confinement étanche 13, qui est constituée, dans le mode de réalisation présenté sur la Figure 1, par un certain nombre de plaques de confinement.

Il y a tout d'abord en position supérieure deux plaques supérieures 14 et 15. Il y a ensuite en position inférieure deux plaques inférieures 16 et 17 ; les plaques 14 et 16 permettent le confinement du module réactionnel 3 notamment au niveau des canaux fluidiques de la zone d'analyse 26, qui sera décrite ultérieurement. Il y a ensuite les plaques supérieures et inférieure ferromagnétiques de confinement 15 et 17 qui, elles, se trouvent positionnées au-dessus dudit module réactionnel 3.

Après la phase de capture, les plaques 15 et 17 sont repositionnées pour fermer le consommable. Seules les micro-organismes 10 restent dans la chambre 9. Les canaux 7 et 8 sont alors utilisés pour faire circuler le tampon d'élution avant la phase de lyse ultrason.

On note que les plaques supérieure 15 et inférieure 17 sont de nature ferromagnétique car elles encadrent la présence d'un certain nombre d'aimants 21 positionnés aux quatre angles du module réactionnel 3 ainsi qu'en son centre. Les aimants 21 assurent, d'une part, la pression des plaques ferromagnétiques 15 et 17 sur les joints toriques 18 et, d'autre part, l'étanchéité après la capture des microorganismes. Ces plaques 15 et 17 permettent d'avoir accès aux billes de capture 6, et d'effectuer la capture des microorganismes 10 présents dans le fluide 2 sur les billes 6. Les plaques 15 et 17 coulissent à la surface du dispositif 1 par l'intermédiaire de glissières 29, situées sur les côtés, elles sont dans le cas présent en forme de queue d'aronde. Le prélèvement d'air terminé, lesdites plaques 15 et 17 sont repositionnées à leur emplacement d'origine par coulissement le long des glissières 29. Le circuit de liquide est alors fermé. Il faut une force de « plaquage » des plaques afin d'assurer une parfaite étanchéité à la fermeture. Cette force est par exemple ferromagnétique, ce qui à l'avantage d'être une force de pression homogène sur toute la surface des plaques concernées.

Entre les cartes principale 4 et secondaire 5 et les plaques supérieure 15 et inférieure 17 l'étanchéité est assuré par un ensemble de joints toriques d'étanchéité 18. Ces joints toriques 18 sont au nombre de trois en position supérieure et de trois également en position inférieure et sont concentriques. Bien entendu, ce nombre est tout à fait non limitatif puisque l'utilisation d'un seul joint torique peut suffire. De plus, d'autres moyens d'étanchéité existent entre les plaques supérieure 14 et inférieure 16 et le reste du dispositif 1. Les moyens utilisés sont notamment un collage de chacune de ces plaques avec une colle ou un adhésif double face sur la carte principale 4. De la même manière la carte secondaire 5 est collée définitivement sur le corps 4 du dispositif. Ce collage est effectué par ultrason, laser ou par voie thermique, il est définitif et permet l'assemblage du dispositif de collecte 1.

On note que la carte principale 4 comporte deux zones où des canaux sont présents. Il y a tout d'abord une zone de stockage référencée 25 à proximité du module réactionnel 3. Il y a également une zone d'analyse 26, qui a été évoquée précédemment. L'ensemble des canaux présents au niveau des zones 25 et 26 permet la gestion fluidique de l'ensemble de la carte. Le canal de la zone de stockage 25 est le réservoir de stockage du liquide réactionnel 12 (tampon d'élution). Les canaux de la zone d'analyse 26 sont les canaux fluidiques de préparation de l'échantillon pour analyse. Ils circulent sur le recto et le verso de la carte. La lecture de fluorescence se fait également dans les canaux de la zone 26. Etant donné que ces zones 25 et 26 ne sont pas l'essence de l'invention, elles ne seront pas exposées plus avant.

De tels types de canaux et leurs fonctions sont mieux expliqués dans une précédente demande de brevet WO-A-2011/033231 déposée sous priorité française du 18 septembre 2009 par la demanderesse. Le lecteur est invité à s'y référer pour une information plus complète.

Après capture des microorganismes 10, les plaques 15 et 17 sont refermées. Le tampon de la chambre 25 est envoyé dans la chambre de capture 9 pour remettre en suspension les microorganismes. Puis la lyse par ultrason est effectuée dans cette même chambre 9 par un moyen extérieur à la carte. Le lysat est ensuite partagé via le tiroir distributeur 22, qui coulisse dans le trou 31, et envoyé dans les canaux de la zone 26 à l'aide des pistons pompes 23, coulissant eux dans les trous 32, pour la préparation à la détection et l'amplification des acides nucléiques 11.

Toutefois il convient de comprendre qu'un certain nombre d'éléments permettent la gestion fluidique et le transfert du liquide réactionnel 12 de compartiment à compartiment grâce à l'intervention du tiroir distributeur 22, ainsi que des pistons pompes 23. Trois de ces pistons pompes 23 sont présents sur la gauche de la carte principale 4, les trois autres étant présents à la droite de cette même carte 4.

Enfin, pour en finir avec la description de la Figure 1, on note la présence d'emplacements dessiccateurs 24 toujours au sein de la carte principale 4. Ces emplacements 24 étant situés à proximité de la zone d'analyse 26, ils contiennent des desséchants permettent d'optimiser la conservation des réactifs intégrés à la carte sous forme de billes de réactif lyophilisé, également appelées « pellets » obtenues par des moyens de lyophilisation, par séchage ou encore par gélification. Les desséchants ne sont pas en contact direct mais à proximité des pellets. Ces pellets contiennent les réactifs nécessaires à l'amplification des acides nucléiques 11.

Le tiroir distributeur 22 gère la distribution des fluides dans le dispositif de collecte 1. De ce fait, il y a plusieurs positions qui assurent :
- le stockage du liquide réactionnel 12,
- le transfert du liquide réactionnel 12 vers la chambre ou moyen de rétention 9 contenant les billes 6 (après capture des microorganismes 10),
- le transfert vers les six canaux de la zone 26 d'un total de 120 µl de volume du lysat, soit 20 µl par canal.

Enfin les pistons pompes 23 sont semblables à des seringues qui permettent d'aspirer et de transférer le liquide réactionnel 12 durant tout le processus. Leur particularité est de fonctionner en cycle d'air clos (aucun air n'est aspiré de l'extérieur), bien qu'une version plus simple avec une aspiration d'air extérieur est envisageable. L'intérêt de ce vase clos est d'éviter au maximum la communication vers l'extérieur et ainsi le risque de contamination croisée de l'intérieur vers l'extérieur et vice versa.

On note sur cette Figure 1, la présence d'une lame de ressort 19 de verrouillage pour les plaques ferromagnétiques 15 et 17. Cette lame 19 est solidarisée au module réactionnel 3 par une vis 20. Ainsi ces plaques amovibles ne doivent pas pouvoir être manipulées de manière non intentionnelle. Cette lame de ressort 19 permet de bloquer et d'empêcher leur ouverture inopinée.

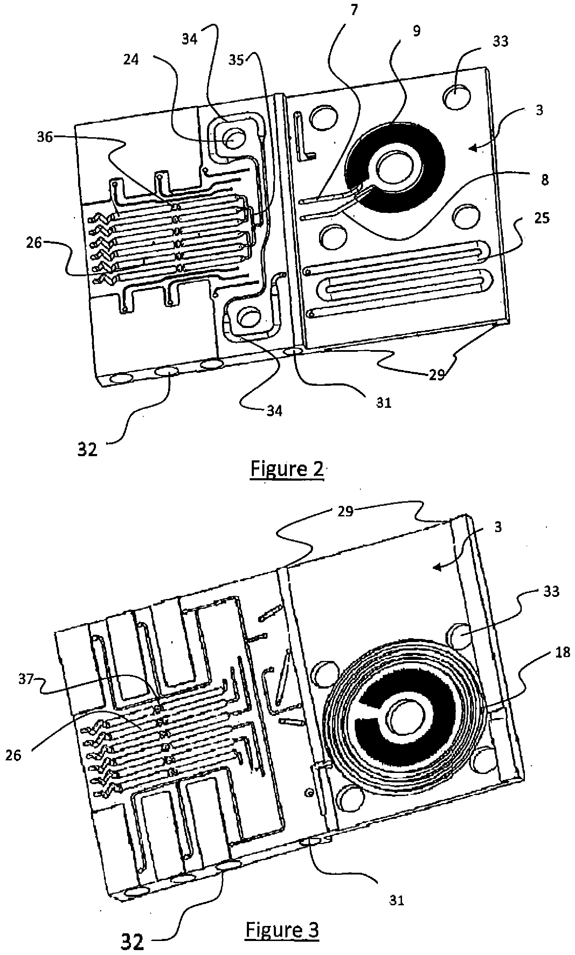

La Figure 2 représente une vue plus détaillée de la carte principale 4. Celle-ci se compose de deux parties d'épaisseur différentes, sur la gauche l'épaisseur est plus importante et comporte la zone d'analyse 26. On note également la présence des emplacements desséchants 24, ainsi que d'un trou 31 pouvant recevoir le tiroir distributeur 22, lui non représenté sur cette figure. Enfin il y a également la présence de six trous 32 pouvant recevoir les six pistons pompe 23, eux aussi non représentés sur cette figure, au sein de la carte principale 4. Sur la partie de faible épaisseur de la carte principale 4, c'est-à-dire sur la droite de cette figure, on note la présence de la zone de stockage 25 du liquide réactionnel 12, après que ledit liquide réactionnel 12 ait :
- remis en suspension les microorganismes 10 capturés,
- permis la lyse grâce à l'agitation des billes 6 par les ultrasons via ce liquide 12,
- transporté les organismes d'intérêt, et
- finalement fourni le milieu réactionnel 12 nécessaire à l'amplification et à la détection des acides nucléiques 11.

La Figure 3 représente la carte secondaire 4. Sur cette partie on note la présence des emplacements pouvant recevoir les joints toriques 18 ainsi que des emplacements 33 pouvant recevoir les aimants 21, joints 18 et aimants 21 sont non présentés sur cette figure.

La Figure 4 montre une vue agrandie d'un détail de la Figure 2, au niveau du moyen de rétention 9 des billes 6 ainsi que des canaux 7 et 8. Les deux extrémités opposées du module en forme de « C » sont connectées fluidiquement aux canaux d'admission et d'évacuation du liquide réactionnel 7 et 8.

Dans le cas présent, le moyen 9 est constitué par une grille 27, mieux représentée sur la Figure 5A ou 5B. On note que l'espace séparant les trous 28 de la grille 27 est faible, par exemple pour des billes de 500 µm, les trous auront un diamètre de 200 à 300 µm et seront séparés les uns des autres par des espaces de 200 à 300 µm, ce qui entraîne que les fluides 2 ou liquides 12 sont forcés au sein de cet espace, ce qui améliore et augmente les points de contact entre les microorganismes, contenant les acides nucléiques d'intérêt, et la surface des billes 6 permettant une meilleure capture desdits acides nucléiques 11. Les billes 6 sont également recouvertes d'une matière facilitant encore plus l'adhésion des microorganismes à la surface des billes. Cette matière est notamment du glycérol dans le cas où le fluide utilisé est un gaz. Ainsi l'utilisation du glycérol fonctionne uniquement dans le cas d'une collecte de microorganismes dans un gaz, notamment l'air. Dans ce cas de figure, l'air passe sur le glycérol et les microorganismes sont capturées sur celui-ci. Le glycérol est ensuite dissous par le tampon réactionnel 12. S'il y a présence de bactéries, elles seront alors lysées dans ce tampon par les ultrasons. Dans le cas de la collecte de microorganismes présents dans de l'eau, cela ne fonctionne pas avec le glycérol qui est dissous par l'eau, et est entrainé hors de la carte. Pour arriver à un fonctionnement correct avec un milieu liquide, il faut utiliser un substitut au glycérol, par exemple un revêtement de polymère qui ne se dissous pas dans l'eau mais uniquement dans un tampon réactionnel spécifique ou permet un "relargage" dans ce tampon spécifique ou ajouter un filtre en aval des billes afin que le fluide entre en contact avec les billes avant de circuler au travers du filtre.

Selon la Figure 5A et 5B, au niveau de la carte principale 4, où est présent la chambre ou moyen de rétention 9, on note la présence de deux conduits référencés 40 et 41. Le conduit 40 permet l'admission du fluide 2 au sein du module 3, fluide 2 qui contient les bactéries à capturer pour analyse ultérieure, alors que le conduit 41 permet l'évacuation de ce même fluide 2 à l'extérieur dudit module 3. Bien entendu, entre l'admission et l'évacuation, le fluide 2 sera passé au sein du module 3 où sont présentes les billes 6 ; c'est à ce niveau que la collecte des microorganismes pourra être effectuée. Si le fluide 2 est introduit et évacué par les conduits 40 et 41, le liquide réactionnel 12 qui peut être constitué par un tampon d'élution ou autre et quant à lui introduit par un canal d'admission 7 au sein du module 3, représenté sur la Figure 4, il est également évacué de ce même module 3 par un canal d'évacuation 8, également représenté sur la Figure 44

En fait ce fluide 2, par exemple de l'air mais qui peut aussi être un liquide différent du liquide réactionnel 12, circule perpendiculairement à la carte. Il coule à travers les grilles 27.

Les Figures 5 à 8 décrivent le protocole de capture d'un microorganisme de manière simplifiée.

Dans la Figure 5A, il est décrit une partie du dispositif 1 selon l'invention, au niveau du traitement du fluide 2, dont on veut extraire les micro-organismes 10, dans lequel il y a :
- des billes 6 présentes dans la cavité, formant moyen de rétention 9,
- une grille supérieure et une grille inférieure 27, percées de plusieurs trous 28, qui confinent les billes 6 au sein du dispositif 1 et donc du moyen de rétention 9 de la carte 4,
- une plaque supérieure ferromagnétique de confinement 15,
- une plaque inférieure ferromagnétique de confinement 17, les plaques 15 et 17 n'étant pas collées à la carte 4 comme le sont les plaques supérieure et inférieure de confinement 14 et 16.
- le canal fluidique non représenté sur cette figure. Selon la Figure 5B, le dispositif 1 est sensiblement identique à la configuration de la Figure 5A, mais les plaque supérieure ferromagnétique de confinement 15 et plaque inférieure ferromagnétique de confinement 17 sont translatées selon F1. Ceci permet au fluide 2 de passer, selon F2, au sein des billes 6, maintenues en position par les grilles 27, selon un mouvement perpendiculaire au canaux pour le ou les liquides réactionnels, non représenté sur la figure. Ce mouvement est effectué par l'intermédiaire des conduits d'admission 40 et d'évacuation 41 du fluide 2 au sein du module 3.

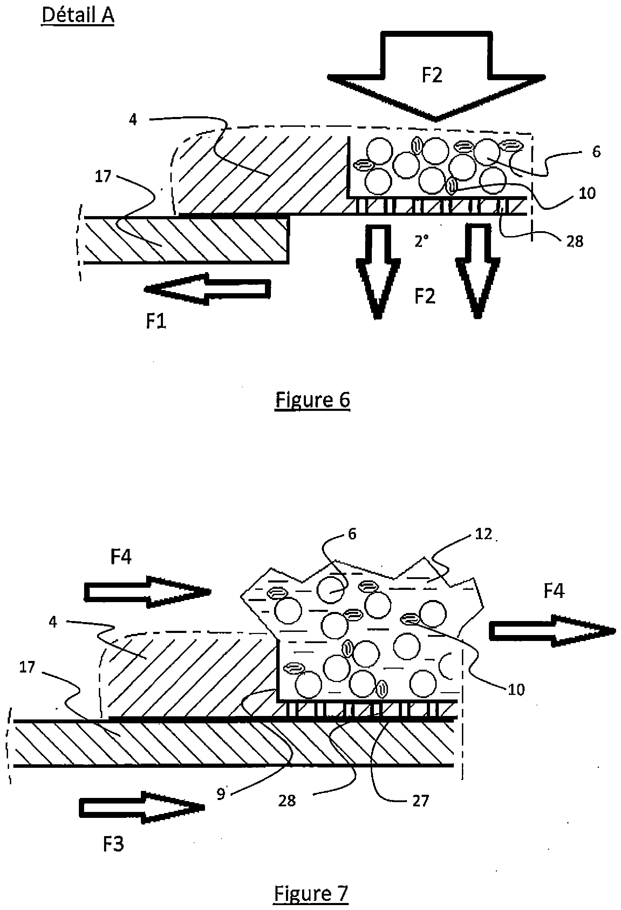

Selon la Figure 6, le détail A de la Figure 5B est mis en avant. Dans cette configuration, les plaques de confinement 15 et 17 amovibles sont enlevées selon F1, et le fluide 2 circule perpendiculairement à l'axe des grilles 27. Les micro-organismes 10 sont capturés sur les billes 6 au sein du moyen de rétention 9.

Dans la Figure 7, les plaques 15 et 17 amovibles sont remises en position fermée par l'intermédiaire d'un coulissement selon F3. Le tampon d'élution 12 est inséré dans la chambre 9 par le canal d'admission 7. Il décroche les micro-organismes 10 des billes 6.

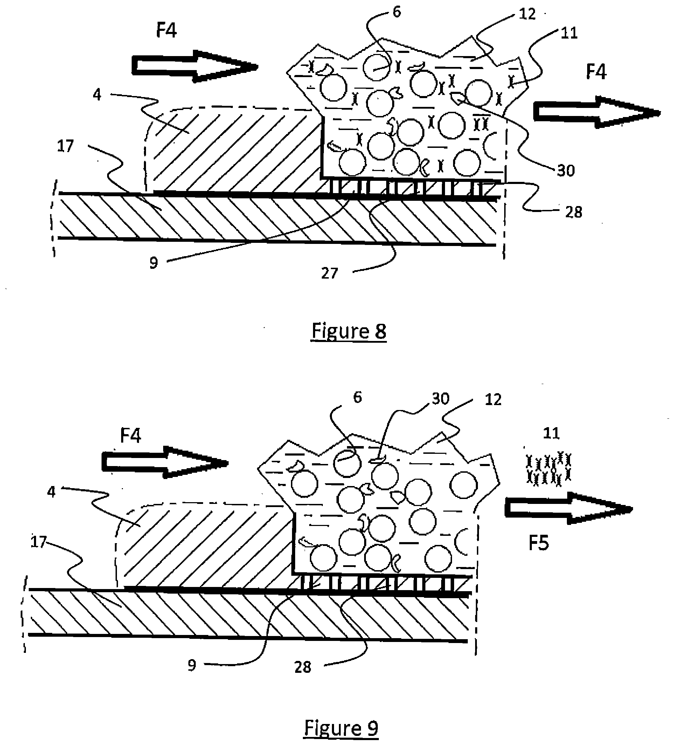

Enfin sur la Figure 8, une fois la chambre 9 remplie, une lyse à ultrasons est effectuée pour lyser les micro-organismes, par exemple des bactéries, 10 et libérer les acides nucléiques 11. Le lysat contenant les acides nucléiques 11 est envoyé dans les canaux via le canal d'évacuation 8, pour préparer l'amplification par remise en suspension des billes de réactif lyophilisé, par détection optique, etc.

Le dispositif permet donc de capturer les micro-organismes 10 notamment de l'air sur des billes de verre glycérolées en faisant circuler le flux d'air à travers un tapis de billes 6. La capture de micro-organismes à travers une surface sèche présente de nombreux avantages, tels que :
- l'absence d'usure du média durant la capture (due à l'évaporation),
- pas d'évaporation durant le stockage,
- très forte concentration des micro-organismes,
- très grande surface développée de capture (due aux microbilles) par rapport à une surface plane d'impaction,
- la surface de capture se sature plus difficilement,
la capture est donc beaucoup plus efficace qu'un système par impaction.

De plus le dispositif est conçu pour :
- offrir une large surface hors plan pour la capture des microorganismes du fluide.
- réduire les pertes de charge et donc l'encombrement de la pompe ; ainsi lors de la capture des microorganismes présents dans l'air, le dispositif est raccordé à une pompe qui aspire l'air à travers la grille de capture 27.
- permettre la reprise des micro-organismes avec un tampon liquide quelconque simplement par pipetage du fait de l'utilisation de canaux fluidiques dans le plan de capture ; ainsi après la capture des microorganismes sur le glycérol, les plaques amovibles 15 et 17 sont refermées. Le tampon de reprise (stockés dans la chambre 25) est poussé dans la chambre contenant les billes. Les microorganismes sont alors pris dans le glycérol, ce dernier se dissolvant dans le tampon, afin que les micro-organismes capturés soient relargués.
- que la lyse (par ultrasons notamment) soit intégrée dans le consommable ou dispositif de collecte de microorganismes 1,
- obtenir un haut rendement de capture avec une faible perte de charge, l'air passant à travers un tapis de billes glycérolés (et non impacté sur un tapis de billes).

Le rendement de capture dépend beaucoup de l'épaisseur du tapis de bille et de la taille des billes mais le rendement de capture est significativement plus important avec une capture en transmission à travers les billes, notamment pour les tailles de particules inférieures à 1 µm. Ce mode capture présente des difficultés techniques tels que :
- la maîtrise de la perte de charge,
- le maintien des billes dans la chambre de capture,
- la reprise des micro-organismes avec un tampon,
- la lyse.

La capture à sec s'effectue sans bullage ni gel aqueux, sans perte d'efficacité dans le temps (> 4 m³ de prélèvement). L'avantage du glycérol est qu'il ne sèche pas. De ce fait, le volume d'air prélevé peut être très important, sans perte notable d'efficacité.
Les captures sur des surfaces aqueuses (gélose, boîte de pétri) ou liquide aqueux (type Coriolis) présentent l'inconvénient de s'assécher au fur et à mesure de la capture. Cet asséchement provoque la diminution du rendement de capture. La capture dans un liquide défini détermine le type d'analyse réalisée en aval de la collecte alors qu'avec une capture à sec les microorganismes peuvent être repris avec n'importe quel tampon.
De plus, la capture sur une surface sèche (billes de verres + glycérol) permet de relarguer les micro-organismes dans un volume très faible de tampon, inférieur à 1 mL voire inférieur à 100 µL. L'avantage de concentrer les microorganismes est de favoriser leur détection. Les systèmes de détection en biologie moléculaire ont une limite de détection de l'ordre de 1 génome équivalent par microlitre (µL).
Avec les dispositifs conventionnels, il faut donc capturer beaucoup plus (de 15 à 30 fois plus soit de 200 à 600 µL) de microorganismes car le volume de capture est de 15 mL (volume important qui dilue les microorganismes).

La lyse mécanique est très efficace et est compatible avec tous les types de procédé biologique, tel que la lyse ultrason dans n'importe quel tampon nécessaire aux étapes suivantes du protocole.
La lyse des micro-organismes capturés est intégrée au dispositif. La technique de lyse est purement mécanique (par opposition à la lyse chimique) et assure ainsi un excellent rendement de lyse et ce quel que soit le tampon de lyse choisi ainsi que le microorganisme à lyser.
Il est ainsi possible d'utiliser le lysat tel quel, pour effectuer ensuite une amplification par exemple NASBA ou une PCR.

Selon une utilisation préférentielle du dispositif 1 proposé par l'invention après que les micro-organismes 10 ont été lysés et que les acides nucléiques 11 ont été repris par le liquide réactionnel ou tampon d'élution 12, le dispositif de collecte 1 permet d'effectuer une amplification ainsi qu'une détection desdits acides nucléiques 11 extraits. Pour ce faire, il y a principalement quatre étapes qui sont réalisées après la lyse.

La première étape consiste à extraire le tampon 12 contenant les acides nucléiques 11 ainsi qu'un certain nombre de résidus 30 de micro-organismes 10, qui sont présents dans le liquide 12 après la lyse, ces résidus n'entrant pas en ligne de compte par la suite dans les analyses qui seront effectuées au niveau de la zone d'analyse 26. Dans cette première étape, le liquide 12 chargé des acides nucléiques 11 et des résidus 30 va sortir du moyen de rétention 9 par l'intermédiaire du canal d'évacuation 8. Au niveau du canal d'évacuation 8, il est par exemple possible d'avoir un filtre qui permet de ne laisser passer que les éléments biologiques d'une taille inférieure ou égale aux acides nucléiques extraits.

Dans une seconde étape ce mélange est envoyé dans une zone permettant l'aliquotage 34, comme cela est bien représenté sur la Figure 2. Le volume ainsi obtenu correspond à 120 µl.

Cet aliquot est ensuite envoyé vers une troisième étape dans une zone de séparation du lysat référencée 35. Sur cette figure, il y a six zones qui sont bien différenciées les unes des autres pour permettre une séparation en six fois 20 µl dans les canaux d'analyse de la zone 26. Dans ces canaux de la zone 26, est effectuée une amplification qui se fait par l'intermédiaire de réactifs permettant l'amplification, tels que des nucléotides, des amorces d'amplification et des sondes de détection, qui se trouvent positionnés en zone intermédiaire 36. On remarque sur la Figure 3 qu'il existe une seconde zone intermédiaire 37 contenant au moins une enzyme (une enzyme si on effectue une PCR, deux si on effectue une TMA et trois si on effectue une NASBA), qui se trouve au recto de la carte principale 4.

La quatrième et dernière étape s'effectue au niveau des canaux de la zone 26, comme cela est bien présenté sur la Figure 3 à la droite de la zone intermédiaire 37 où la détection peut être réalisée.

L'ensemble de ces mouvements est donc réalisé par l'intermédiaire du tiroir distributeur 22 et des pistons pompes 23 qui, par leur coulissement non représentés sur les figures, permettent de diriger les liquides des différentes zones vers d'autres zones qui vont permettre l'amplification des acides nucléiques 11 et leur détection subséquente. Le tiroir distributeur 22 et les pistons pompes 23 possèdes des joints, bien représentés sur la Figure 1, respectivement 38 et 39 qui assurent l'étanchéité du système.

### EXEMPLE 1 : Rendement de capture des micro-organismes (avec un compteur de particule) en fonction de la taille des billes et de l'épaisseur du tapis de billes,

Pour un même dispositif voici le graphique du rendement de capture en fonction de la taille des billes et de l'épaisseur du lit de bille.

### 1. A. Mode opératoire :

Les plaques coulissantes 15 et 17 sont ouvertes durant toute l'expérience.
Pour cette expérience, on utilise un compteur de particules qui évalue la quantité de particules par mètre cube (m3) d'air aspiré. Les particules détectées sont classées dans différentes catégories de taille compris entre 0,3 µm, 0,5 µm, 1 µm et 5 µm). Trois cycles de mesures sont effectués :
1 : Mesure référence à vide sans dispositif.
2 : Mesure d'évaluation avec le dispositif en amont du compteur de particules.
3 : Une seconde mesure de référence.

### 1.B. Expérience :

Les mesures de référence à vide sont moyennées. Le rendement de capture est calculé par le rapport entre mesure avec et sans dispositif.

Les résultats sont indiqués dans le Tableau 1 ci-dessous :

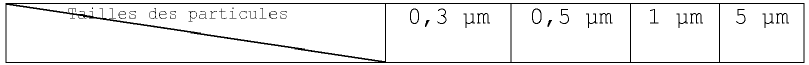

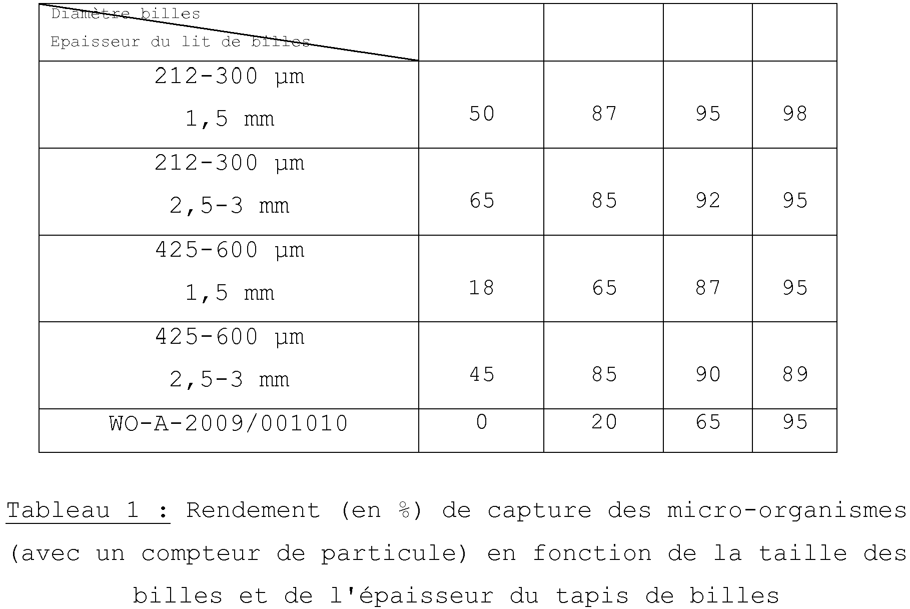

### 1.C. Analyse :

Pour un même dispositif 1, le tableau 1 montre que le rendement de capture est très efficace quelles que soient la taille des billes et l'épaisseur du lit de bille par rapport à l'état de la technique constitué par le dispositif décrit par le document PCT. Les particules d'une taille inférieure à 0,5 µm ne semblent pas donner un rendement suffisant même si cela reste bien supérieur à l'état de la technique (minimum de 18% de rendement contre zéro). Dans une taille comprise entre 0,5 et 5 µm les résultats sont bons (toujours supérieurs à 65% et même si on exclut le cas à 65%, toujours supérieurs à 85%). Toutes les épaisseurs de lit de billes testées sont acceptables.

### EXEMPLE 2 : Optimisation de la perte de charge de chacun des dispositifs testés :

### 2.A. Mode opératoire :

Chaque dispositif est placé sur un banc d'essai qui permet de mesurer la perte de charge en fonction du débit d'air. L'objectif est de trouver le meilleur compromis entre l'efficacité de capture du dispositif 1 et sa perte de charge. Une perte de charge élevée signifie une consommation électrique élevée du dispositif de prélèvement (non favorable à une application portable).

### 2.B. Expérience :

Le Tableau 2 est un tableau d'aide au design du dispositif de capture des microorganismes. Il compare le rendement de capture des particules (par gamme de taille : 0,5-1 µm, 1-5 µm, 5-25 µm) à la perte de charge en pression (mBar) du dispositif. L'énergie nécessaire pour pomper un certain volume d'air à travers le dispositif est proportionnel à la perte de charge (pour un même débit). Pour une application de pompe portative il est nécessaire de trouver le bon compromis entre le rendement de capture et la perte de charge (voir Tableau 2).

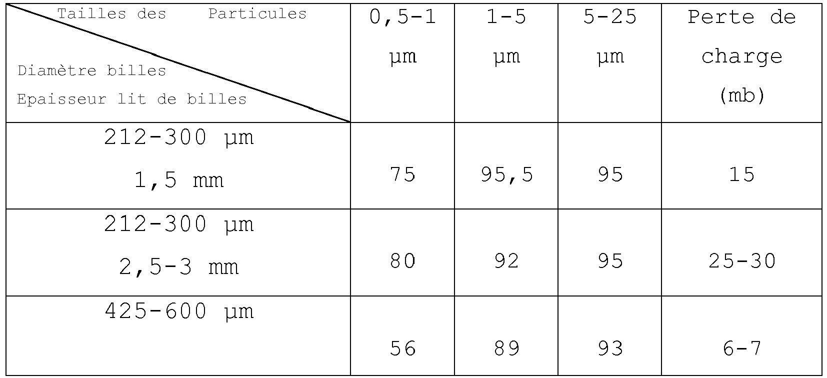

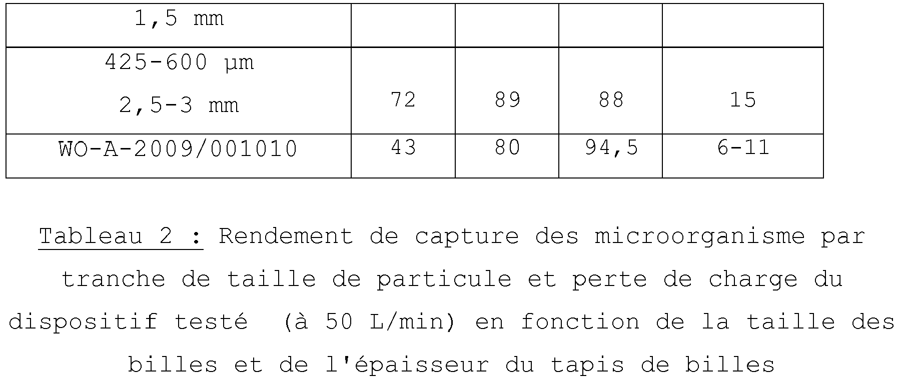

### 2.C. Analyse :

A la lumière de ce tableau et en comparaison avec la demande de brevet WO-A-2009/001010, constituant l'état de la technique, on peut voir que le dispositif selon l'invention, quel que soit l'épaisseur du lit de billes et quel que soit le diamètre des billes utilisés, est toujours plus performant que la solution proposée par l'état de la technique. De plus, avec des billes de 425 à 600 µm et une épaisseur de lit de 1,5 mm les résultats sont les plus efficaces pour capturer les particules que le dispositif du brevet WO-A-2009/001010 et ce pour une perte de charge en pression équivalente.

### EXEMPLE 3 : Remplissage et vidange des dispositifs :

### 3.A. Mode opératoire :

Seule la chambre 9 de billes 6 est testée. L'entrée de la chambre de bille (pleine de bille mais sans liquide) est connectée à un raccord fluidique et une pipette. Le liquide est poussé dans la chambre de bille jusqu'à son remplissage. Une fois remplie, celle-ci est vidée de la même manière.

### 3.B. Expérience :

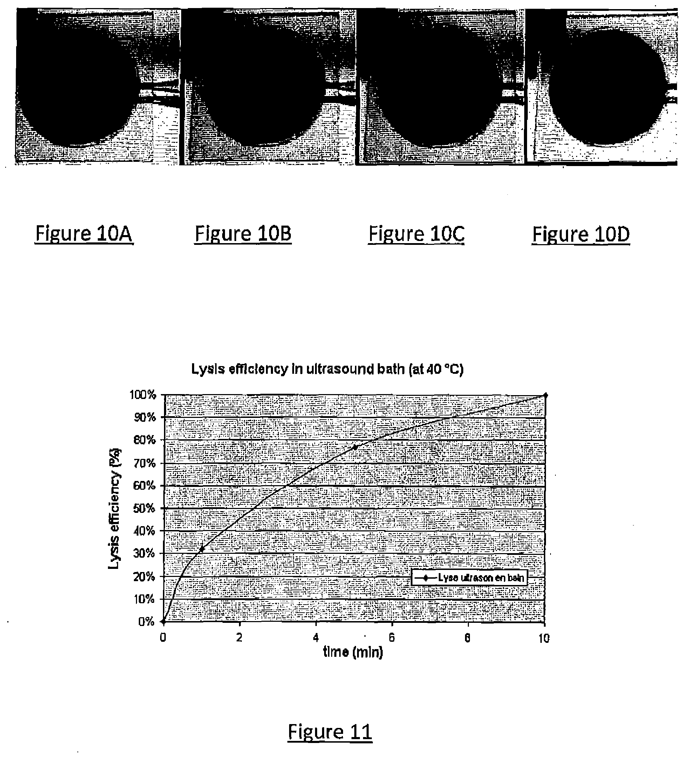

Les Figures 10A à 10D montre le remplissage de la chambre 9 où les micro-organismes 10 sont capturés. On injecte 500 µL au niveau du canal d'admission 7 et on arrive à récupérer 200 µL au niveau du canal d'évacuation 8.

### 3.C. Résultats :

Le remplissage s'effectue parfaitement.

### EXEMPLE 4 : Lyse des micro-organismes :

### 4.A. Mode opératoire :

Un dispositif 1 composé d'une chambre de lyse est rempli avec un tampon 12 contenant des *Staphylococcus epidermidis.* Le dispositif est soumis à des ultrasons, via une sonotrode, afin de lyser les micro-organismes. L'expérience est répétée avec un temps de lyse de 0, 1, 5 et 10 minutes. Le rendement de lyse est évalué par pousse et comptage des lysats sur boite de Petri. L'objectif de cette expérience est de trouver une interface convenable pour la transmission des ultrasons lors de l'étape de lyse.

### 4.B. Expérience :

En relation avec la Figure 11, un même design de dispositif de chambre à billes est utilisé avec quelques configurations d'interface, soit :
- 1^{ère} configuration : avec des éléments en plastique remplaçant les plaques ferromagnétiques 15 et 17 (sonotrode en contact direct avec un dispositif modifié selon l'invention),
- 2^{ème} configuration : avec une couche de silicone (présente entre la sonotrode et le dispositif selon l'invention),
- 3^{ème} configuration : avec des éléments métalliques faisant office de plaques ferromagnétiques 15 et 17 ajoutées sur dispositif.

Les trois dispositifs sont testés comme précédemment et pour deux types de diamètre de bille (Voir Tableau 3).

**Tableau 3 : Efficacité de la lyse par ultrasons**

| Dispositif | Standard | | Insert métal | | Couche silicone |
|---|---|---|---|---|---|
| Diamètre bille de verre (µm) | 212-300 | 425-600 | 212-300 | 425-600 | 212-300 |
| Efficacité de lyse (%) | 90-100 | 50 | 100 | 98 | 40-55 |

### 4.C. Résultats :

Les résultats sont assez bons pour l'ensemble des tests effectués. Toutefois la solution utilisant les inserts métalliques donnent de bons résultats sensiblement identiques pour les deux types de billes.

### EXEMPLE 5 : Lyse et amplification NASBA sans purification de micro-organismes (S. epidermidis):

### 5.A. Mode opératoire :

Un dispositif est rempli de tampon contenant des bactéries S. *epidermidis* ainsi qu'un contrôle interne constitué d'une bactérie différente de cette bactérie testée. La lyse est effectuée dans le dispositif selon la 3^{ème} configuration décrite à l'exemple 4. Le Lysat est ensuite extrait du dispositif et analysé.

### 5.B. Expérience :

Les courbes de détection sont présentées dans les Tableaux 4 & 5 ci-dessous.

| | | | | | | | |
|---|---|---|---|---|---|---|---|
| Référence (gamme de contrôle) (eq. CFU/µl) | 0 | 0,1 | 1 | 10 | 100 | 1000 | 10000 |
| Résultat | Négatif | Positif | Positif | Positif | Positif | Positif | Positif |

| | | | | | |
|---|---|---|---|---|---|
| Echantillons testés (eq. CFU/µl) | 0 | 0,2 | 2 | 20 | 200 |
| Résultat | Négatif | Positif | Positif | Positif | Positif |

Cette expérience montre l'efficacité du dispositif décrit à lyser les microorganismes et à les détecter grâce à une analyse NASBA. Les microorganismes sont injectés dans le tampon 12 avant l'expérience (une concentration différente de microorganisme à chaque expérience). Les volets métalliques de la carte sont fermés. La partie contenant les billes est remplie de tampon 12 (qui contient différentes concentrations de microorganismes). Le dispositif est placé sur une sonde ultrason pour effectuer la lyse. A la fin de l'étape de lyse, le tampon 12 est collecté manuellement et analysé en NASBA en dehors de la carte. Ces résultats sont à comparer à une gamme de contrôle.

En première intention l'interprétation des résultats est binaire : Positif signifie qu'il y a détection du microorganisme et Négatif signifie que le microorganisme n'est pas détecté.

### 5.C. Analyse :

On remarque dans les tableaux ci-dessus que les échantillons testés sont marqués comme positif pour toutes les concentrations supérieures à 0,2 CFU/µl.

### REFERENCES

1. Dispositif de collecte de microorganismes 10
2. Fluide
3. Module réactionnel
4. Carte principale
5. Carte secondaire
6. Billes
7. Canal d'admission du liquide réactionnel 12 du module 3
8. Canal d'évacuation du liquide réactionnel 12 du module 3
9. Moyen de rétention des billes 6 au sein du module 3
10. Microorganismes présents dans le fluide 2
11. Acides nucléiques présents dans les microorganismes 10
12. Liquide réactionnel
13. Enceinte de confinement étanche constituée des plaques 14 à 17
14. Plaque supérieure de confinement du dispositif 1
15. Plaque supérieure ferromagnétique coulissante de confinement du dispositif 1
16. Plaque inférieure de confinement du dispositif 1
17. Plaque inférieure ferromagnétique coulissante de confinement du dispositif 1
18. Joints torique d'étanchéité entre l'enceinte 13 et les cartes 4 et 5
19. Lame ressort de verrouillage des plaque ferromagnétiques 15 et 17
20. Vis de fixation de la lame ressort 19
21. Aimants
22. Tiroir distributeur
23. Pistons pompes
24. Emplacements dessiccateurs
25. Zone de stockage du liquide réactionnel 12
26. Zone d'analyse sur le recto et le verso de la carte
27. Grilles supérieure et inférieure
28. Trous de la grille 27
29. Glissières
30. Résidus de microorganismes présents dans le liquide 12 après la lyse
31. Trou recevant le tiroir distributeur 22
32. Trous recevant les pistons pompes 23
33. Trous traversants des cartes 4 et 5 recevant les aimants 21
34. Zone d'aliquotage
35. Zone de séparation
36. Première zone intermédiaire contenant une enzyme
37. Seconde zone intermédiaire contenant une enzyme
38. Joints sur le tiroir distributeur 22
39. Joints sur les pistons pompes 23
40. Conduit d'admission du fluide 2 au sein du module 3
41. Conduit d'évacuation du fluide 2 au sein du module 3
F1. Coulissement des plaques supérieure 15 et inférieure 17 ferromagnétiques permettant l'ouverture pour le passage du fluide 2
F2. Mouvement du fluide 2 passant au travers des billes 6
F3. Coulissement des plaques supérieure 15 et inférieure 17 ferromagnétiques permettant la fermeture pour le passage du liquide 12
F4. Mouvement du liquide 12 passant au travers des billes 6
F5 Extraction des acides nucléiques 11

## Revendications

1. Dispositif de collecte de microorganismes (1) contenus dans un fluide (2), le dispositif comportant :
• un premier module réactionnel (3) renfermant un lit de billes (6) apte à immobiliser les microorganismes,
• au moins un conduit d'admission (40) du fluide (2) permettant l'entrée d'un flux de fluide à l'intérieur du premier module réactionnel (3),
• au moins un conduit d'évacuation (41) du fluide (2) permettant la sortie du flux dudit fluide ayant traversé l'intérieur dudit premier module réactionnel (3),
• des moyens de rétention (9) des billes (6) à l'intérieur du premier module réactionnel (3),
• au moins un canal d'admission (7) d'au moins un liquide réactionnel (12),
• au moins un canal d'évacuation (8) d'au moins un liquide réactionnel (12),
les canaux d'admission (7) et d'évacuation (8) du liquide réactionnel (12) et les conduits d'admission (40) et d'évacuation (41) du fluide (2) étant positionnés de la manière suivante au niveau du premier module (3) réactionnel :
• les conduits d'admission (40) et d'évacuation (41) du fluide se faisant face tout en enserrant le premier module réactionnel (3), afin de maximiser les contacts entre le fluide (2) et les billes (6),
• les canaux pour le ou les liquides réactionnels (12) étant placés respectivement à deux extrémités opposées du premier module réactionnel (3), et
• les canaux (7 et 8) pour le ou les liquides réactionnels (12) sont positionnés selon un plan, et les conduits d'admission (40) et d'évacuation (41) du fluide sont positionnés selon un axe, et que l'axe est sensiblement perpendiculaire au plan.

2. Dispositif, selon la revendication 1, **caractérisé par le fait qu'**il comprend une enceinte de confinement étanche (13) isolant fluide et liquides réactionnels de l'extérieur.

3. Dispositif selon l'une quelconque des revendications 1 ou 2, **caractérisé par le fait que**, si le fluide est un gaz, l'ensemble de billes (6) est constitué de billes recouvertes de glycérol.

4. Dispositif selon l'une quelconque des revendications 1 ou 2, **caractérisé par le fait que**, si le fluide est un liquide, le premier module réactionnel (3) contient un filtre s'étendant sensiblement dans le plan formé par les canaux pour le ou les liquides réactionnels (12), le conduit d'admission (40) étant situé d'un côté du filtre avec l'ensemble de billes (6) et le conduit d'évacuation (41) étant situé de l'autre côté dudit filtre en l'absence de billes.

5. Dispositif, selon l'une quelconque des revendications 1 à 4, **caractérisé par le fait que** le premier module réactionnel (3) a une forme de « C » si on le coupe :
• selon le plan formé par le(s) canaux d'admission (7) et d'évacuation (8) du liquide réactionnel (12), et
• perpendiculairement à l'axe formé par le(s) conduit(s) d'admission (40) et le(s) conduit(s) d'évacuation (41) du fluide.

6. Dispositif, selon la revendication 5, **caractérisé par le fait que** le premier module réactionnel a une forme de quadrilatère si on le coupe selon un rayon passant par le centre du « C » et coupant ledit « C ».

7. Dispositif, selon l'une quelconque des revendications 5 ou 6, **caractérisé par le fait que** le(s) canal (canaux) d'admission (40) du liquide réactionnel (12) est situé à une extrémité du « C » de la forme du premier module réactionnel (3), et que le(s) canal (canaux) d'évacuation (41) du liquide réactionnel (12) est situé à l'autre extrémité du « C ».

8. Dispositif, selon l'une quelconque des revendications 1 à 7, **caractérisé par le fait que** les billes (6) ont un diamètre compris entre 200 et 600 µm et que les moyens de rétention (9) desdites billes (6) à l'intérieur du premier module réactionnel (3) sont constitués de grilles (27), dont les pores ont un diamètre inférieur aux billes (6) et compris entre 100 et 500 µm.

9. Dispositif, selon l'une quelconque des revendications 1 à 8, **caractérisé par le fait qu'**il comprend un deuxième module réactionnel permettant le traitement des microorganismes collectés, le deuxième module réactionnel contenant :
a. des moyens de séparation des résidus cellulaires sans intérêt ou inhibiteurs, tels que protéines, membranes, et/ou
b. des moyens d'amplification des acides nucléiques collectées et séparés des inhibiteurs, et/ou
c. des moyens de détection des amplicons ainsi générés.

10. Procédé de collecte de microorganismes contenus dans un fluide au moyen d'un dispositif de collecte de microorganismes (1) selon l'une quelconque des revendications 1 à 9, **caractérisé en ce qu'**il consiste à :
a. introduire un fluide suspecté de contenir des microorganismes via au moins un conduit d'admission (40) du fluide,
b. traverser un lit de billes (6) où les microorganismes sont immobilisés,
c. évacuer le fluide débarrassé desdits microorganismes via au moins un conduit d'évacuation (41) dudit fluide,
d. introduire au moins un liquide réactionnel (12) via au moins un canal d'admission de liquide(s) réactionnel(s),
e. traverser le lit de billes (6) où les microorganismes sont immobilisés afin de mettre lesdits microorganismes en suspension, et
f. évacuer ce liquide transportant les microorganismes via au moins un canal d'évacuation de liquide(s) réactionnel(s).

11. Procédé, selon la revendication 10, **caractérisé en ce que**, lorsque le liquide traverse le lit de billes (6), lesdites billes (6) sont agitées par un moyen d'agitation extérieur et entrent en contact ce qui permet de lyser les membranes des microorganismes, rendant les acides nucléiques accessibles et prêts à être évacués.

12. Procédé, selon la revendication 11, **caractérisé en ce que** l'agitation des billes (6) est réalisée par l'utilisation d'ultrasons.

13. Procédé, selon l'une quelconque des revendications 10 à 12, **caractérisé en ce que** le liquide chargé de tout ou partie des microorganismes est évacué vers un deuxième module réactionnel du dispositif (1) permettant le traitement des microorganismes collectés, le traitement consistant à :
a. séparer les résidus cellulaires sans intérêt ou inhibiteurs, tels que protéines, membranes, et/ou
b. amplifier les acides nucléiques collectées, et/ou
c. détecter les amplicons ainsi générés.

14. Procédé selon la revendication 13, **caractérisé en ce que** le liquide est filtré entre l'évacuation via au moins un canal d'évacuation de liquide(s) réactionnel(s) et le deuxième module réactionnel, qui permet l'élimination des inhibiteurs et/ou l'amplification et/ou la détection.

15. Procédé, selon l'une quelconque des revendications 10 à 14, **caractérisé en ce que** :
a. le fluide testé est constitué par un gaz,
b. le gaz traverse le lit de billes, billes qui sont recouvertes de glycérol, où les microorganismes sont immobilisés, et
c. l'on introduit au moins un liquide réactionnel afin de liquéfier le glycérol et de mettre lesdits microorganismes en suspension.

16. Procédé, selon l'une quelconque des revendications 10 à 14, **caractérisé en ce que** :
a. le fluide testé est constitué par un liquide,
b. le liquide testé traverse le lit de billes puis un filtre avant d'être évacué, débarrassé des microorganismes qu'il contenait, les microorganismes sont immobilisés, et
c. l'on introduit au moins un liquide réactionnel afin de mettre lesdits microorganismes en suspension.

## Patentansprüche

1. Vorrichtung zum Sammeln von Mikroorganismen (1), die in einem Fluid (2) enthalten sind, wobei die Vorrichtung umfasst:
• ein erstes Reaktionsmodul (3), das ein Kugelbett (6) umschließt, das geeignet ist, die Mikroorganismen zu immobilisieren,
• mindestens eine Zuführleitung (40) für das Fluid (2), die das Eintreten eines Fluidstroms in das Innere des ersten Reaktionsmoduls (3) ermöglicht,
• mindestens eine Abführleitung (41) für das Fluid (2), die das Austreten des Stroms des Fluids ermöglicht, welches das Innere des ersten Reaktionsmoduls (3) durchströmt hat,
• Rückhaltemittel (9) zum Zurückhalten der Kugeln (6) im Inneren des ersten Reaktionsmoduls (3),
• mindestens einen Zuführkanal (7) für mindestens eine Reaktionsflüssigkeit (12),
• mindestens einen Abführkanal (8) für mindestens eine Reaktionsflüssigkeit (12),
wobei der Zuführkanal (7) und der Abführkanal (8) für die Reaktionsflüssigkeit (12) und die Zuführleitungen (40) und Abführleitungen (41) für das Fluid (2) am ersten Reaktionsmodul (3) folgendermaßen angeordnet sind:
• die Zuführleitung (40) und die Abführleitung (41) für das Fluid liegen einander gegenüber und umfassen das Reaktionsmodul (3), um die Kontakte zwischen dem Fluid (2) und den Kugeln (6) zu maximieren,
• die Kanäle für die Reaktionsflüssigkeit(en) (12) sind jeweils an zwei entgegengesetzten Enden des ersten Reaktionsmoduls (3) platziert und
• die Kanäle (7 und 8) für die Reaktionsflüssigkeit(en) (12) sind entlang einer Ebene angeordnet, und die Zuführleitung (40) und die Abführleitung (41) für das Fluid sind entlang einer Achse angeordnet, und die Achse verläuft im Wesentlichen senkrecht zu der Ebene.

2. Vorrichtung nach Anspruch 1, **dadurch gekennzeichnet, dass** sie eine dichte Umschließungskammer (13) umfasst, die Fluid und Reaktionsflüssigkeiten vom Außenbereich isoliert.

3. Vorrichtung nach einem der Ansprüche 1 oder 2, **dadurch gekennzeichnet, dass**, wenn das Fluid ein Gas ist, sämtliche Kugeln (6) aus mit Glycerol überzogenen Kugeln bestehen.

4. Vorrichtung nach einem der Ansprüche 1 oder 2, **dadurch gekennzeichnet, dass**, wenn das Fluid eine Flüssigkeit ist, das erste Reaktionsmodul (3) ein Filter enthält, das sich im Wesentlichen in der durch die Kanäle für die Reaktionsflüssigkeit (en) (12) gebildeten Ebene erstreckt, wobei die Zuführleitung (40) mit sämtlichen Kugeln (6) auf einer Seite des Filters und die Abführleitung (41) ohne Kugeln auf der anderen Seite des Filters gelegen ist.

5. Vorrichtung nach einem der Ansprüche 1 bis 4, **dadurch gekennzeichnet, dass** das erste Reaktionsmodul (3) die Form eines "C" hat, wenn man es durchschneidet:
• entlang der Ebene, die durch den oder den Zuführkanal (7) und den Abführkanal (8) für die Reaktionsflüssigkeit (12) gebildet wird, und
• senkrecht zu der Achse, die durch die Zuführleitung(en) (40) und die Abführleitung(en) (41) für das Fluid gebildet wird.

6. Vorrichtung nach Anspruch 5, **dadurch gekennzeichnet, dass** das erste Reaktionsmodul die Form eines Vierecks hat, wenn man es entlang eines Radius durchschneidet, der durch den Mittelpunkt des "C" verläuft und das "C" durchschneidet.

7. Vorrichtung nach einem der Ansprüche 5 oder 6, **dadurch gekennzeichnet, dass** der Zuführkanal oder die Zuführkanäle (40) für die Reaktionsflüssigkeit (12) an einem Ende des "C" der Form des ersten Reaktionsmoduls (3) gelegen ist (sind) und dass der Abführkanal oder die Abführkanäle (41) für die Reaktionsflüssigkeit (12) am anderen Ende des "C" gelegen ist (sind).

8. Vorrichtung nach einem der Ansprüche 1 bis 7, **dadurch gekennzeichnet, dass** die Kugeln (6) einen Durchmesser zwischen 200 und 600 µm aufweisen und dass die Rückhaltemittel (9) zum Zurückhalten der Kugeln (6) im Inneren des ersten Reaktionsmoduls (3) aus Gittern (27) bestehen, deren Poren einen Durchmesser aufweisen, der kleiner als die Kugeln (6) ist und zwischen 100 und 500 µm liegt.

9. Vorrichtung nach einem der Ansprüche 1 bis 8, **dadurch gekennzeichnet, dass** sie ein zweites Reaktionsmodul umfasst, das die Behandlung der gesammelten Mikroorganismen ermöglicht, wobei das zweite Reaktionsmodul enthält:
a. Mittel zur Separation der Zellreste, die nicht von Interesse sind oder Inhibitoren sind, wie Proteine, Membranen, und/oder
b. Mittel zur Amplifikation der gesammelten und von den Inhibitoren separierten Nukleinsäuren und/oder
c. Mittel zur Detektion der so erzeugten Amplikons.

10. Verfahren zum Sammeln von Mikroorganismen, die in einem Fluid enthalten sind, mittels einer Vorrichtung zum Sammeln von Mikroorganismen (1) nach einem der Ansprüche 1 bis 9, **dadurch gekennzeichnet, dass** es in Folgendem besteht:
a. Einführen eines Fluids, das verdächtig ist, Mikroorganismen zu enthalten, durch mindestens eine Zuführleitung (40) für das Fluid,
b. Durchströmen eines Kugelbetts (6), in dem die Mikroorganismen immobilisiert werden,
c. Abführen des von den Mikroorganismen befreiten Fluids durch mindestens eine Abführleitung (41) für das Fluid,
d. Einführen mindestens einer Reaktionsflüssigkeit (12) durch mindestens einen Zuführkanal für Reaktionsflüssigkeit(en),
e. Durchströmen des Kugelbetts, in dem die Mikroorganismen immobilisiert sind, um die Mikroorganismen zu suspendieren, und
f. Abführen dieser die Mikroorganismen transportierenden Flüssigkeit durch mindestens einen Abführkanal für Reaktionsflüssigkeit(en).

11. Verfahren nach Anspruch 10, **dadurch gekennzeichnet, dass** die Kugeln (6), wenn die Flüssigkeit das Kugelbett (6) durchströmt, mit einer externen Schütteleinrichtung geschüttelt werden und in Kontakt gelangen, was es ermöglicht, die Membranen der Mikroorganismen zu lysieren und so die Nukleinsäuren zugänglich und abführbereit zu machen.

12. Verfahren nach Anspruch 11, **dadurch gekennzeichnet, dass** das Schütteln der Kugeln (6) unter Einsatz von Ultraschall ausgeführt wird.

13. Verfahren nach einem der Ansprüche 10 bis 12, **dadurch gekennzeichnet, dass** die mit allen oder einem Teil der Mikroorganismen gefüllte Flüssigkeit zu einem zweiten Reaktionsmodul der Vorrichtung (1) abgeführt wird, das die Behandlung der gesammelten Mikroorganismen ermöglicht, wobei die Behandlung in Folgendem besteht:
a. Separieren der Zellreste, die nicht von Interesse sind oder Inhibitoren sind, wie Proteine, Membranen, und/oder
b. Amplifikation der gesammelten Nukleinsäuren und/oder
c. Detektieren der so erzeugten Amplikons.

14. Verfahren nach Anspruch 13, **dadurch gekennzeichnet, dass** die Flüssigkeit zwischen dem Abführen durch mindestens einen Abführkanal für Reaktionsflüssigkeit(en) und dem zweiten Reaktionsmodul gefiltert wird, was die Elimination der Inhibitoren und/oder die Amplifikation und/oder die Detektion ermöglicht.

15. Verfahren nach einem der Ansprüche 10 bis 14, **dadurch gekennzeichnet, dass**:
a. das getestete Fluid aus einem Gas besteht,
b. das Gas das Kugelbett aus mit Glycerol überzogenen Kugeln durchströmt, in dem die Mikroorganismen immobilisiert werden, und
c. man mindestens eine Reaktionsflüssigkeit einführt, um das Glycerol zu verflüssigen und die Mikroorganismen zu suspendieren.

16. Verfahren nach einem der Ansprüche 10 bis 14, **dadurch gekennzeichnet, dass**:
a. das getestete Fluid aus einer Flüssigkeit besteht,
b. die getestete Flüssigkeit das Kugelbett und dann ein Filter durchströmt, bevor sie befreit von den zuvor enthaltenen Mikroorganismen abgeführt wird, die Mikroorganismen immobilisiert werden, und
c. man mindestens eine Reaktionsflüssigkeit einführt, um die Mikroorganismen zu suspendieren.

## Claims

1. Collecting device (1) configured to collect microorganisms contained in a fluid (2), said collecting device comprising:
- a first reaction module (3) containing a set of beads (6) configured to capture microorganisms,
- at least one admission duct (40) of the fluid (2) configured to allow a stream of fluid to enter the first reaction module,
- at least one evacuating duct (41) of the fluid configured to allow the exit of the stream of said fluid having being through the inside of the first reaction module (3),
- retaining elements (9) that retain the beads (6) within the first reaction module(3),
- at least one admission channel (7) configured to allow admission of at least one reaction liquid (12), and
- at least one evacuating channel (8) configured to allow exit of the reaction liquid (12),
wherein the admission channel (7) and evacuating channel (8) and the admission duct (40) and evacuating duct (41) being positioned at the first reaction module as follows :
- the admission (40) and evacuating (41) ducts face one another while enclosing the first reaction module(3) in to order to maximize contacts between the fluid (2) and the beads (6),
- the admission (7) and evacuating (8) channels are positioned respectively at two opposite ends of the first reaction module (3) and
- the admission (7) and evacuating (8) channels are positioned in a plane, the admission (40) and evacuation (41) ducts are arranged along an axis, said axis being perpendicular to the plane.

2. Collecting device according to claim 1, comprising an airtight confinement enclosure (13) isolating reaction fluids and reaction liquids from outside.

3. Collecting device according to claim 1 or 2, wherein if the fluid is a gas, the set of beads (6) consists of beads coated with glycerol.

4. Collecting device according to claim 1 or 2, wherein if the fluid is a liquid, the first reaction module (3) comprises a filter extending substantially in the plane formed by the channels for the reaction liquid(s) (12), the admission duct (40) being positioned on one side of the filter with the set of beads (6) and the evacuating duct (41) being positioned on the other side of said filter without the set of beads.

5. Collecting device according to any of the claims 1 to 4, wherein the first reaction module (3) has a "C" shape :
- according to the plane formed by admission channel (7) and evacuation channel (8) of reaction liquid (12), and
- perpendicularly to the axis formed by admission duct(s) (40) and evacuation duct(s) (41).

6. Collecting device according to claim 5, wherein the first reaction module has a quadrilateral shape if it is cut along a radius passing through the center of the "C" and cutting through said "C".

7. Collecting device according to claims 5 or 6, wherein the admission channel (7) is connected to one end of the "C" shape of the cavity, and the evacuating channel (8) is connected to the other end of the "C" shape.

8. Collecting device according to any of the claims 1 to 7, wherein the beads have a diameter in the range from 200 and 600 µm, the retaining elements (9) of the beds (6) inside the first reaction module (3) include grids (27) having pores presenting a diameter smaller than the diameter of the beads and in the range from 100 to 500 µm.

9. Collecting device according to any of the claims 1 to 8, further comprising a second reaction module configured to allow the treatment of microorganisms collected, the second reaction module comprising:
a. separation means of cellular residues without interest or inhibitors, such as proteins, membranes, and/or
b. amplification means of nucleic acids collected and separated from inhibitors and/or
c. detection means of amplicons generated.

10. Method for collecting microorganisms contained in a fluid with the collecting device according to claims 1 to 9, the method comprising the steps :
a. introducing a fluid suspected to contain microorganisms via the at least one admission duct (40) of the fluid,
b. passing through a set of beads (6) where microorganisms are immobilized
c. evacuating the fluid cleared from said microorganisms via the at least one evacuating duct (41); and
d. introducing the at least one reaction liquid (12) via at least one reaction liquid admission channel,
e. passing through a set of beads (6) where microorganisms are immobilized so that the microorganisms are in suspension,
f. evacuating the reaction liquid transporting said microorganisms via at least one evacuating channel.

11. Collecting method according to claim 10, wherein when the reaction liquid passes through the set of beads (6), the beads (6) being agitated by external agitation means and coming in contact with each other to lyse membranes of the microorganisms, making nucleic acids of said microorganisms accessible and ready to be evacuated.

12. Collecting method according to claim 11, wherein the agitation of the beads (6) is performed by ultrasounds.

13. Collecting method accordingto any of the claims 10 to 12,, wherein the liquid containing all or a part of microorganisms , is evacuated towards a second reaction module of the device (1) allowing treatment of microorganisms collected, the treatment consisting in :
- separating cellular residues without interest or inhibitors such as proteins, membranes, and/or
- amplifying nucleic acids collected, and/or
- detecting amplicons generated.

14. Collecting method according to claim 13, wherein the liquid is filtered between evacuation via at least one evacuation channel of reaction liquid and the second reaction module, in order to allow elimination of inhibitors and/or amplification and/or detection.

15. Collecting method according to any one of the claims 10 to 14, wherein
a. The fluid tested is a gas,
b. The gas passes through a set of beads where microorganisms are immobilized, the beads being covered by glycerol,
c. At least one reaction liquid is introduced in order to liquefy glycerol and to put microorganisms in suspension.

16. Collecting method according to any one of the claims 10 to 14, wherein
a. The fluid tested is a liquid,
b. The liquid passes through a set of beads where microorganisms are immobilized, and a filter before being evacuated while being cleared of microorganisms,
c. At least one reaction liquid is introduced in order to put microorganisms in suspension.
